# EUROPÄISCHE PATENTSCHRIFT

(11) **EP 3 359 517 B1**
(45) Veröffentlichungstag und Bekanntmachung des Hinweises auf die Patenterteilung: **25.09.2019**
(21) Anmeldenummer: 16778375.2
(22) Anmeldetag: 07.10.2016
(51) Int. Cl.: C07C 45/28, C07C 45/54, C07C 45/79, C07C 45/82, C07C 49/587

(54) **VERFAHREN ZUR REINIGUNG VON CYCLOHEXADEC-8-EN-1-ON**
METHOD FOR THE PURIFICATION OF CYCLOHEXADEC-8-EN-1-ON
PROCEDE DESTINE AU NETTOYAGE DE CYCLOHEXADEC -8-EN-1-ON

(30) Priorität: 08.10.2015 EP 15188864
(43) Veröffentlichungstag der Anmeldung: 15.08.2018
(73) Patentinhaber: BASF SE, 67056 Ludwigshafen am Rhein (DE)
(72) Erfinder: THRUN, Frauke, 68163 Mannheim (DE); TELES, Joaquim Henrique, 67165 Waldsee (DE); WERNER, Albert, 78343 Bishop (US); PELZER, Ralf, 37699 Fürstenberg (DE)
(86) Internationale Anmeldenummer: PCT/EP2016/074016
(87) Internationale Veröffentlichungsnummer: WO 2017/060437

(56) Entgegenhaltungen:
- EP-A2- 1 264 594
- WO-A1-2010/086314
- DE-A1-102011 082 464

## Beschreibung

Die vorliegende Erfindung betrifft ein neuartiges Verfahren zur Reinigung von Cyclohexadec-8-en-1-on, ein Verfahren zur Herstellung von Cyclohexadec-8-en-1-on und Cyclopentadecenonen, die damit hergestellten Stoffe und Stoffgemische und deren Verwendung als Aromastoff, insbesondere als Riechstoff, sowie Aromastoffzusammensetzungen und Mittel, enthaltend diese Gemische.

### Hintergrund der Erfindung

In der Parfümindustrie besteht ein ständiger Bedarf an neuen Riechstoffen, die sich als Riechstoffkompositionen oder parfümierten Artikeln eignen.

Insbesondere besteht ein Bedarf an moschusartigen Riechstoffen und Riechstoffkompositionen. Hierunter ist ein Geruch zu verstehen, der dem natürlich vorkommenden Moschusduft ähnlich ist.

Cyclohexadec-8-en-1-on (als *cis*/*trans*-Isomerengemisch) ist ein kommerzieller Riechstoff (Globanon® von Symrise). *Trans-* und *cis*-Cyclopentadec-8-en-1-on finden bereits Erwähnung in US 5,936,100 und in Fürstner, A. et al, Synthesis 1997, 792. Die Verbindungen wurden ausgehend von Heptadec-1,16-dienon mittels einer Ruthenium-katalysierten Ringschlussalkenmetathese hergestellt.

Globanon® wird technisch aus Cyclohexadeca-1,9-dien (CHDD, EP-A-1 288 181) hergestellt über eine zweistufige Synthese:

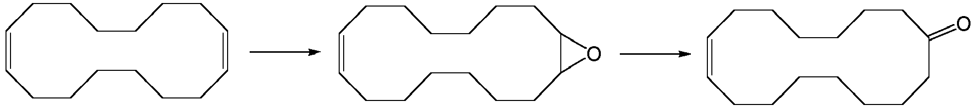

Weil beide Stufen mit Probleme behaftet sind, bestand der Wunsch eine einfachere einstufige Synthese zu entwickeln.

Aus dem Stand der Technik (WO 2012/084673) ist die einstufige Oxidation von Cyclohexadeca-1,9-dien mit N₂O bekannt. Dabei entstehen als Hauptprodukt (E/Z)-Cyclohexadec-8-en-1-on sowie als Nebenprodukte verschiedene Cyclopentadecenylcarbaldehyde. Eine destillative Trennung des Gemisches aus Cyclohexadec-8-en-1-on und Cyclopentadecenylcarbaldehyd ist nur mit einem hohen Ausbeuteverlust an Cyclohexadec-8-en-1-on möglich. Eine Trennung des Gemisches aus Cyclohexadec-8-en-1-on und Cyclopentadecenylcarbaldehyd muss aber erfolgen, da sonst Cyclohexadec-8-en-1-on mit einer Fehlnote (Geruch nach Plastik) behaftet ist.

In der EP-A-1 264 594 wird die Verwendung von *cis-* und *trans*-Cyclopentedec-7-en-1-on als Beimischung in einer Formulierung zur Inhibierung der Melaninsynthese erwähnt. Die EP-A 216 185 erwähnt, dass die Verbindungen der *cis*-7- und 8-Cyclopentadecen-1-one ausgehend von *cis*-16-Oxabicyclo-[13.1.0]-hexadec-8-en über eine Säure oder Lewis-Säure katalysierte Meerwein-Umlagerung hergestellt werden können, ohne hierzu jedoch eine experimentellen Beleg zu liefern. Ferner wird die Brauchbarkeit der beiden Verbindungen als Duftstoff postuliert. Die tatsächlichen olfaktorischen Eigenschaften der jeweiligen (E/Z)-7- bzw. -8-Cyclopentadecenone wurden jedoch in der Literatur bisher noch nicht beschrieben.

Die Cu-katalysierte oxidative Decarbonylierung ist bekannt und vielfältig publiziert, Beispiele finden sich: a) Tetrahedron Letters 1969, 12, 985; US3496197; b) 1995 Tetrahedron Letters 4641, c) Org. Lett. 2010, 2630, d) Bioorg. Med. Chem. Lett. 2013, 23, 5949, e) Chin. Chem. Lett. 2014, 25, 771.

Es bestand daher die Aufgabe eine Methode zu finden, wie man die Aldehyde, die als Nebenprodukte bei der Globanon®-Synthese durch N₂O Oxidation gebildet werden, wirtschaftlich abtrennen kann. Da eine destillative Trennung alleine nicht zum Ziel führt, ist eine chemische Abtrennung bevorzugt. Chemische Abtrennungen von aldehydische Nebenprodukte aus Ketone, die durch N₂O-Oxidation hergestellt werden, sind ebenfalls aus dem Stand der Technik (WO2008/000752) bekannt. Diese Methoden, die auf einer Aldolkondensation basieren, können zwar sehr effektiv den Aldehyd in leicht abtrennbare Hochsieder überführen, führen aber dazu, dass pro mol Aldehyd ein mol des Ketons geopfert wird. Es bestand also insbesondere die Aufgabe, eine verbesserte Methode zu finden, die nicht zu Ketonverlusten führt und aus den Aldehyden möglichst ein weiteres Wertprodukt erzeugt. WO 2010/086314 A1 offenbart ein Verfahren zur Herstellung von Cyclododecanon.

### Zusammenfassung der Erfindung

Überraschenderweise wurde obige Aufgabe insbesondere durch Bereitstellung der in den Ansprüchen definierten Verfahren zur Reinigung von Globanon gelöst. Die Cyclopentadecenylcarbaldehyde werden einer Cu-katalysierten oxidativen Decarbonylierung unterworfen, wobei sie zu Cyclopentadecenonen abgebaut werden, die dann leicht destillativ von Cyclohexadec-8-en-1-on getrennt werden können. Die Cyclopentadecenone selbst sind wertvolle Moschusriechstoffe.

### Detaillierte Beschreibung der Erfindung

### a) Allgemeine Definitionen:

Werden keine anderen Angaben gemacht, so umfassen die hierin verwendeten chemischen Formelnamen, jegliche stereoisomerer Form der Verbindung sowie jeglichen Mischungen dieser stereoisomeren Formen.

"Cyclohexadecenon" steht insbesondere für "Cyclohexadec-8-en-1-on", umfasst (E)-Cyclohexadec-8-en-1-on und (Z)-Cyclohexadec-8-en-1-on in stereoisomerenreiner Form oder als Gemisch von E- und Z-Isomeren

"Cyclopentadecenon", umfasst Cyclopentade-8-en-1-on und/oder Cyclopentadec-7-en-1-on und insbesondere, (E)-Cyclopentade-8-en-1-on und/oder (E)-Cyclopentadec-7-en-1-on sowie (Z)-Cyclopentade-8-en-1-on und/oder (Z)-Cyclopentadec-7-en-1-on, sowie Stereoisomerengemische davon.

"Cyclopentadecenylcarbaldehyd" umfasst Cyclopentade-8-en-1-on und/oder Cyclopentadec-7-en-1-on und insbesondere , (E)-Cyclopentadec-8-enyl-1-carbaldehyd und/oder (E)-Cyclopentadec-7-enyl-1-carbaldehyd sowie (Z)-Cyclopentadec-8-enyl-1-carbaldehyd und/oder (Z)-Cyclopentadec-7-enyl-1-carbaldehyd, sowie Stereoisomerengemische davon.

"Cyclohexadecadien", steht insbesondere für Cyclohexadeca-1,9-dien, insbesondere der (E/E), (Z/Z), (E/Z) oder (Z/E) Form von Cyclohexadeca-1,9-dien, sowie Stereoisomerengemische davon.

"Erfindungsgemäß hergestellten Riechstoffe" umfassen insbesondere obige "Cyclohexadecenone" und "Cyclopentadecenone" sowie korrespondierende Stoffgemische davon.

Eine "Aromachemikalie" ist ein Oberbegriff für Verbindungen die als "Riechstoff" und/oder als "Geschmacksstoff" einsetzbar sind.

Als "Riechstoff" sind im Sinne der vorliegenden Erfindung natürliche oder synthetische Stoffe mit Eigengeruch zu verstehen.

Als "Geschmacksstoff" sind im Sinne der vorliegenden Erfindung natürliche oder synthetische Stoffe mit Eigengeschmack zu verstehen.

Der "Geruch" oder die "olfaktorische Wahrnehmung" ist im Sinne der vorliegenden Erfindung die Interpretation der Sinneserregungen, die von den Chemorezeptoren der Nase oder anderen Geruchsorganen an das Gehirn eines Lebewesens geliefert werden. Der Geruch kann folglich eine Sinneswahrnehmung der Nase von Riechstoffen sein, die beim Einatmen erfolgt. Die Luft dient hierbei als Geruchsträger.

Als "Duft" ist im Sinne der vorliegenden Erfindung ein wohlriechender Geruch zu verstehen. Entsprechendes gilt für einen erfindungsgemäßen "Duftstoff".

Ein "Parfüm" ist im Sinne der vorliegenden Erfindung ein Gemisch aus Riechstoffen und Träger, wie insbesondere einem Alkohol.

Eine "Parfümzusammensetzung" ist im Sinne der vorliegenden Erfindung ein Parfüm, welches unterschiedliche Mengen harmonisch aufeinander abgestimmter Einzelkomponenten enthält. Die Eigenschaften der Einzelbestandteile werden genutzt, um in der Kombination ein neues Gesamtbild zu schaffen, wobei die Charakteristika der Ingredienzen in den Hintergrund treten, ohne jedoch unterdrückt zu werden.

Ein "Parfümöl" ist im Sinne der vorliegenden Erfindung ein konzentriertes Gemisch aus mehreren Riechstoffen, die z.B. in alkoholischen Lösungen zur Parfümierung verschiedener Produkte gebraucht werden.

Ein "Duftthema" ist im Sinne der vorliegenden Erfindung die vorherrschende Duftnote in einer Riechstoffzusammensetzung.

Die "Kopfnote" ist im Sinne der vorliegenden Erfindung die erste Phase des Duftablaufs eines Parfüms. Sie spielt die ausschlaggebende Rolle beim ersten Eindruck, beim Öffnen des Flakons und beim Auftragen des Parfüms auf die Haut. Die Aufgabe der Kopfnote ist es, Interesse für das Parfüm allgemein zu wecken und für Aufmerksamkeit zu sorgen. Deshalb ist ein außergewöhnlicher Charakter häufig wichtiger als eine ausgefeilte Harmonie. Die Kopfnote wird naturgemäß von leichtflüchtigen Riechstoffen bestimmt.

"Modifizieren" bedeutet im Sinne der vorliegenden Erfindung, das Grundthema einer Riechstoffzusammensetzung mit zusätzlichen oder anderen Akkorden und Geruchsnuancen zu versehen.

"Akkorde" entstehen im Sinne der vorliegenden Erfindung durch das Zusammenfügen verschiedener Riechstoffe, die sich somit zu neuen Geruchsbildern vereinigen. Die Anzahl der eingesetzten Riechstoffe kann von zwei bis zu hundert Verschiedene reichen.

Eine "organoleptisch/sensorisch wirksame Menge" im Sinne der vorliegenden Erfindung ist die Menge eines Riechstoffes, die ausreicht, um anregend auf ein Sinnesorgan beziehungsweise anregend auf einen sensorischen Rezeptor zu wirken.

(E/Z) steht für (E und/oder Z) und bezeichnet grundsätzlich, soweit nicht anders angegeben, Mischungen umfassend sowohl die stereoisomere E-Konfiguration als auch die entsprechende Z- Konfiguration, aber auch die stereoisomerenreinen E- und Z-Formen einer Verbindung.

### b) Spezielle Ausführungsformen der Erfindung

Vorliegende Erfindung betrifft insbesondere folgende Gegenstände:
1. Verfahren zur Herstellung von Cyclohexadec-8-en-1-on, in stereoisomerenreiner Form oder als Gemisch von E- und Z-Isomeren, insbesondere als Gemisch von E- und Z-Isomeren, und Cyclopentadecenon, insbesondere Cyclopentade-8-en-1-on und/oder Cyc-lopentadec-7-en-1-on, jeweils in stereoisomerenreiner Form oder als Gemisch von E- und Z-Isomeren, insbesondere als Gemisch von E- und Z-Isomeren, umfassend die folgenden Schritt:
   i) Umsetzung von Cyclohexadeca-1,9-dien, in stereoisomerenreiner Form oder als Gemisch von E- und Z-Isomeren (E/E, Z/Z, E/Z oder Z/E), mit N₂O unter Erhalt eines ersten Reaktionsgemisches (RG1), umfassend Cyclohexadec-8-en-1-on und Cyclopentadecenylcarbaldehyde; insbesondere Cyclopentadec-7/8-enyl-1 - carbaldehyde, insbesondere Cyclopentadec-8-enyl-1-carbaldehyde und Cyclopentadec-7-enyl-1-carbaldehyde (jeweils in stereoisomerenreiner Form oder als Gemisch von E- und Z-Isomeren),
   ii) Umsetzung von RG1 mit Sauerstoff in Gegenwart eines Cu-(II)-Katalysators, wobei die enthaltenen Cyclopentadecenylcarbaldehyde durch oxidative Decarbonylierung zu einem oder mehreren korrespondierenden Cyclopentadecenonen, insbesondere Cyclopentadec-8-en-1-on und Cyclopentadec-7-en-1-on (jeweils in stereoisomerenreiner Form oder als Gemisch von E- und Z-Isomeren), überführt werden, wobei man ein zweites Reaktionsgemisch (RG2) erhält; und
   iii) Isolierung (und insbesondere teilweise oder vollständige Auftrennung) der gebildeten Cyclohexadec-8-en-1-one und Cyclopentadecenone, insbesondere der unten angegebenen Verbindungen der Formeln I bis **VI** aus RG2.
   Die oxidative Decarbonylierung der Stufe ii) erfolgt vorzugsweise in Gegenwart einer katalytischen Menge eines vorzugsweise homogenen, Cu-(II)-Katalysators und molekularem reinem Sauerstoff, Magerluft oder vorzugsweise Luft, insbesondere getrockneter, filtrierter Umgebungsluft.
2. Verfahren nach Ausführungsform 1, wobei man aus RG1 nicht umgesetztes Cyclohexadeca-1,9-dien, vorzugsweise destillativ, abtrennt, wobei man ein Reaktionsgemisch RG1a erhält, umfassend Cyclohexadec-8-en-1-on (z.B. in einem Anteil von, mind. 75% wie z.B. 75 bis 85%) und Cyclopentadecenylcarbaldehyde (z.B. in einem Anteil von mind. 5%, wie z.B. 5 bis 10%), wobei RG1a insbesondere weniger als 1% Cyclohexadeca-1,9-dien enthält; und wobei man nicht umgesetztes Cyclohexadeca-1,9-dien gegebenenfalls in die Umsetzung nach Stufe i) zurückführt.
   Die %-Angaben betreffen jeweils die durch GC-Analyse ermittelten Flächen-% und entsprechen in etwa den Gew.-% Anteilen, bezogen auf den Feststoffgehalt der untersuchten Mischung.
3. Verfahren nach Ausführungsform 2, wobei man RG1a, vorzugsweise destillativ, reinigt, wobei man eine erste Fraktion (F1), angereichert an Cyclohexadec-8-en-1-on, insbesondere mit weniger als 1%, wie z.B. weniger als 0,5 oder 0,1% (z.B. 1.3 - 2.1 mmol) Cyclopentadecenylcarbaldehyde, und wenigstens eine zweite Fraktion (F2), angereichert an Cyclopentadecenylcarbaldehyde, insbesondere enthaltend Cyclohexadec-8-en-1-on (wie z.B. in einem Anteil von 20 bis 80%, insbesondere etwa 30 bis 60%, wie z.B. 50%) und wenigsten 10%, insbesondere wenigstens 20, bevorzugt wenigstens 30% oder mehr, wie z. B. 30 bis 70, oder 35 bis 60 oder 40 bis 50% (z.B. 30 - 100 g Gemisch pro Fraktion) Cyclopentadecenylcarbaldehyde, erhält.
   Die %-Angaben betreffen jeweils die durch GC-Analyse ermittelten Flächen-% und entsprechen in etwa den Gew.-% Anteilen, bezogen auf den Feststoffgehalt der untersuchten Mischung.
4. Verfahren nach einer der vorhergehenden Ausführungsformen, wobei man in Stufe ii) RG1, RG1a oder F2, insbesondere RG1a oder F2, oder eine Mischung davon einsetzt.
5. Verfahren nach einer der vorhergehenden Ausführungsformen, wobei man in Stufe iii) ein Reaktionsgemisch RG2a, erhalten aus der oxidativen Decarbonylierung (Stufe ii) der Fraktion F2 einsetzt.
6. Verfahren nach einer der vorhergehenden Ausführungsformen, wobei der Umsatz der Cyclopentadecenylcarbaldehyde in Stufe ii) mindestens 90%, wie z.B. 91 bis 100% oder 93 bis 98%, bezogen auf die eingesetzte Menge an Carbaldehyden beträgt.
7. Verfahren nach einer der vorhergehenden Ausführungsformen, wobei Cyclohexadec-8-en-1-on und aus der oxidative Decarbonylierung entstandene Cyclopentadecenone in RG2 oder RG2a voneinander destillativ getrennt werden.
8. Verfahren nach einer der vorhergehenden Ausführungsformen, wobei das Cyclohexadec-8-en-1-on als Stereoisomerengemisch von (E)- und (Z)-Cyclohexadec-8-en-1-on in Stufe i) anfällt.
9. Verfahren nach einer der vorhergehenden Ausführungsformen, wobei die in Stufe i) gebildeten Cyclopentadecenylcarbaldehyde ausgewählt sind unter (E/Z)-Cyclopentadec-8-enyl-1-carbaldehyd, (E/Z)-Cyclopentadec-7-enyl-1-carbaldehyd, einzelnen Stereoisomeren davon und Mischungen solcher Stereoisomere.
10. Verfahren nach einer der vorhergehenden Ausführungsformen, wobei das in Stufe ii) gebildete Cyclopentadecenon ausgewählt ist unter (E/Z)-Cyclopentadec-8-en-1-on, (E/Z)-Cyclopentadec-7-en-1-on, einzelnen Stereoisomeren davon und Mischungen solcher Stereoisomere (der Formeln I bis IV).
11. Verfahren nach einer der vorhergehenden Ausführungsformen, wobei die oxidative Decarbonylierung zusätzlich in Gegenwart einer Base erfolgt.
   Die im erfindungsgemäßen Verfahren beispielsweise eingesetzte Base ist beispielsweise ausgewählt unter Diazabicycloalkanen, wie z. B. Diazabicyclooctan (DABCO), Diazabicycloundecen (DBU), Diazabicyclononan (DBN) tertiären Aminen wie Trimethylamin, Triethylamin, Triisopropylamin, Diisopropylethylamin oder Tripropylamin, N,N-Dimethylpiperazin, N-Methylpyridin, N-Methylpyrrolidon, Quinulidin und dergleichen. Vorzugsweise wird DBU eingesetzt.
12. Verfahren nach einer der vorhergehenden Ausführungsformen, wobei die oxidative Decarbonylierung in Abwesenheit oder in Gegenwart eines Lösungsmittels, wie z.B. in Gegenwart eines organischen Lösungsmittel erfolgt, insbesondere eines organischen Lösungsmittels, worin der Katalysator gelöst ist.
   Als nicht limitierende Beispiele für organische Lösungsmittel sind zu nennen: polare, aprotische Lösungsmittel wie Dimethylformamid (DMF), Hexamethylphosphoramid (HMPA), Dimethylsulfoxid (DMSO), Tetramethylharnstoff und Dimethylacetamid, sowie Mischungen davon. Weitere geeignete organische Lösungsmittel, welche alleine oder in Kombination mit obigen aprotischen organischen Lösungsmitteln einsetzbar sind, sind Alkanole, wie beispielsweise Methanol, Ethanol, Propanol oder Butanole, wie *tert*-Butanol sowie Tetrahydrofuran, Dioxan oder Benzol. Vorzugsweise wird DMF eingesetzt
   In einer alternativen Ausführungsform erfolgt die Umsetzung im wesentlich ohne Zugabe von Lösungsmittel, vorzugsweise in einem lösungsmittelfreien Reaktionsansatz. Das Gemisch aus Reaktanden und Katalysator kann dazu vorzugsweise erwärmt werden, wie z.B. auf eine Temperatur 30 bis 70 oder 40 bis 60 °C.
13. Verfahren nach einer der vorhergehenden Ausführungsformen, wobei man den Katalysator in situ bildet, indem man zu einem Cu-(II)-Salz einen Liganden, einen insbesondere zweizähnigen Liganden, vorzugsweise Diamin-Liganden, gibt.
   Das erfindungsgemäß eingesetzte Cu-(II)-Salz ist beispielsweise ausgewählt unter Cu-(II)-acetat, -formiat, -sulfat, -chlorid oder-nitrat, Vorzugsweise wird Cu(OAc)₂ eingesetzt.
   Geeignete Komplexliganden sind insbesondere zweizähnige Kupfer-komplexierende Amin-Liganden, wie N, N, N-, N-Tetramethylethylendiamin (TMEDA), 1,10-Phenantrolin und 2,2-Bipyridyl. Vorzugsweise wird TMEDA eingesetzt.
14. Verfahren nach einer der vorhergehenden Ausführungsformen, wobei die oxidative Decarbonylierung bei einer Temperatur im Bereich von 10 bis 70, insbesondere 30 bis 60 °C, über eine Dauer von 0,1 bis 40, insbesondere 1 bis 30 h , vorzugsweise 2 bis 7 h, vor allem 3 bis 5 h, wie z.B. etwa 4 h, und bei einem Druck von 1 bis 10 , insbesondere 1 bis 2 bar durchgeführt wird. Eine Durchführung des Verfahrens im Vakuum, wie z.B. bei einem Unterdruck im Bereich von 0,001 bis 0,99 bar, oder insbesondere 0,01 bis 0,5 oder 0,1 bis 0,25 bar ist ebenfalls denkbar.
15. Verfahren nach einer der vorhergehenden Ausführungsformen, wobei das in Stufe ii) eingesetzte, RG1, RG1a oder F2, neben dem (E/Z)-Cyclohexadec-8-en-1-on, (E/Z)-Cyclopentadec-8-enyl-1-carbaldehyd oder eine Mischung aus (E/Z)-Cyclopentadec-8-enyl- und (E/Z)-Cyclopentadec-7-enyl-1-carbaldehyd oder einzelner Stereoisomere davon enthält.
16. Verfahren nach einer der vorhergehenden Ausführungsformen, wobei man als Cyclopentadecenon wenigstens eine Verbindung, ausgewählt unter (E/Z)-Cyclopentadec-8-en-1-on und (E/Z)-Cyclopentadec-7-en-1-on oder einzelner Stereoisomere davon erhält.
17. Verfahren nach Ausführungsform 16, wobei man wenigstens eine Verbindung ausgewählt unter Verbindungen der folgenden Formeln **I, II, III** und **IV** 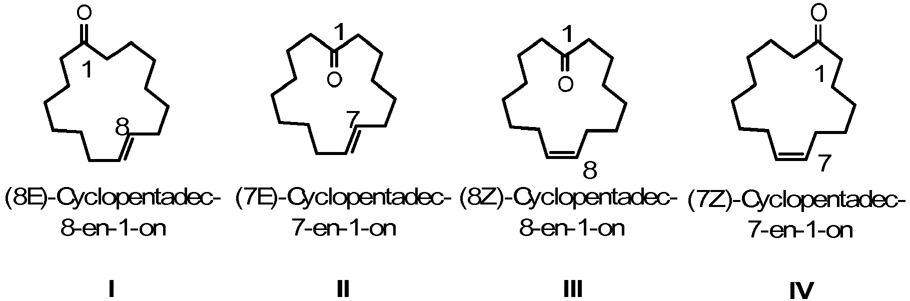 erhält.
18. Verfahren nach einer der vorhergehenden Ausführungsformen, wobei die Isolierung in Stufe iii) eine Trennung von RG2 in wenigstens eine Fraktion F3 enthaltend Cyclohexadec-8-en-1-on und wenigstens eine Fraktion F4 enthaltend wenigstens ein Cyclopentadecenon (**I-IV**)**,** durch Destillation und/oder Chromatographie umfasst.
19. Stoff oder Stoffgemisch erhältlich nach einem Verfahren nach einer der vorhergehenden Ausführungsformen, wobei der Stoff oder das Stoffgemisch ausgewählt ist unter Fraktionen F1, F3 und F4, oder daraus isolierten Einzelverbindungen, gegebenenfalls in stereoisomerenreiner Form; insbesondere Stoffgemisch ausgewählt ist unter Fraktionen F1, F3 und F4 .
20. Verwendung wenigstens eines Stoffes oder Stoffgemisches nach Ausführungsform 19; insbesondere eines Stoffgemischs ausgewählt ist unter Fraktionen F1, F3 und F4, als Aromachemikalie, insbesondere als Riechstoff.
21. Verwendung nach Ausführungsform 20 in Mitteln, ausgewählt unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.
22. Verwendung nach einer der Ausführungsformen 20 und 21 zur Erzeugung einer Moschusnote in einer Riechstoffzusammensetzung.
23. Verwendung nach Ausführungsform 22, zum Vermitteln, Modifizieren und/oder Verstärken einer Moschusduftnote in einer Riechstoffzusammensetzung durch Beimischen einer sensorisch wirksamen Menge wenigstens eines Stoffes oder ein Stoffgemisches gemäß der Definition in einer der Ausführungsformen 19.
24. Riechstoffzusammensetzung, enthaltend wenigstens einen Stoff oder ein Stoffgemisch gemäß der Definition in Ausführungsform 19, insbesondere eine Stoffgemisch ausgewählt ist unter Fraktionen F1, F3 und F4, oder hergestellt nach einem der Verfahren gemäß den Ausführungsformen 1 bis 18.
25. Zusammensetzung nach Ausführungsform 21, enthaltend den Stoff oder das Stoffgemisch in einem Gewichtsanteil von 0,01 bis 99,9 Gew.-% ,1 bis 80 Gew.-%, 2 bis 50 Gew.-%, 3 bis 25 oder 5 bis 15 Gew.-%, bezogen auf das Gesamtgewicht der Zusammensetzung.
26. Mittel enthaltend wenigstens einen Stoff oder ein Stoffgemisch gemäß der Definition in Ausführungsform 19, insbesondere ein Stoffgemisch ausgewählt ist unter Fraktionen F1, F3 und F4, oder hergestellt nach einem der Verfahren gemäß den Ausführungsformen 1 bis 18.
27. Mittel nach Ausführungsform 26, enthaltend den Stoff oder das Stoffgemisch in einem Gewichtsanteil von 0,01 bis 99,9 Gew.-%, 1 bis 80 Gew.-%, 2 bis 50 Gew.-%, 3 bis 25 oder 5 bis 15 Gew.-%, bezogen auf das Gesamtgewicht der Zusammensetzung.
28. Mittel nach Ausführungsform 26 oder 27, ausgewählt unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.
29. Verfahren zur Reinigung von Cyclohexadec-8-en-1-on, wobei man ein wie in einem der Verfahrensansprüche 1 bis 3 definiertes Gemisch RG1a oder F2 einer oxidative Decarbonylierung, definiert wie in einer der Ausführungsformen 1 oder 11 bis 14 unterzieht und Cyclohexadec-8-en-1-on von den gebildeten oxidierten Decarbonylierungsprodukten, insbesondere destillativ und / oder chromatographisch trennt.

### c) Weiter Ausgestaltungen der Erfindung

### c1) Riechstoffzusammensetzungen:

Einem weiteren Aspekt zufolge werden die "erfindungsgemäß hergestellten Riechstoffe" d.h. Cyclohexadecenone und Cyclopentadeceneone, oder Mischungen davon, wie oben definiert, in stereoisomerenreiner Form oder als Gemisch von wenigstens 2 Stereoisomeren, insbesondere zwecks effizienterer Handhabung und Dosierung, auch als Riechstoffmischungen mit Verdünnungs- oder Lösungsmitteln eingesetzt. Hierbei wird der Anteil der Riechstoffe, bezogen auf die Summe von Riechstoffen und Lösungsmittel, in Gew.-% angegeben.

### Lösungsmittel:

Ein "Lösungsmittel" dient im Sinne der vorliegenden Erfindung der Verdünnung der erfindungsgemäß zu verwendenden Riechstoffe oder der erfindungsgemäßen Riechstoffzusammensetzung, ohne eigene riechende Eigenschaften zu besitzen. Manche Lösungsmittel haben zugleich fixierende Eigenschaften.

Die erfindungsgemäß hergestellten Riechstoffe, ein oben definiertes Stoffgemisch aus mehreren Verbindungen/Isomeren davonkann zu 0,1 bis 99 Gew-% einem Verdünnungs- oder Lösungsmittel beigemischt werden. Bevorzugt sind mindestens 40 Gew.-%ige Lösungen, weiter bevorzugt mindestens 50 Gew.-%ige Lösungen, weiterhin bevorzugt mindestens 60 Gew.-%ige Lösungen, weiter bevorzugt mindestens 70 Gew.-%ige Lösungen, insbesondere bevorzugt mindestens 80 Gew.-%ige Lösungen, weiterhin insbesondere bevorzugt mindestens 90 Gew.-%ige Lösungen, vorzugsweise in olfaktorisch akzeptablen Lösungsmitteln.

Bevorzugte olfaktorisch akzeptable Lösungsmittel sind Ethanol, Isopropanol, Dipropylenglycol (DPG), Propylenglycol, 1,2-Butylenglycol, Glycerin, Diethylenglycolmonoethylether, Diethylphthalat (DEP), Isopropylmyristat (IPM), Triethylcitrat (TEC), Benzylbenzoat (BB) und Benzylacetat. Hierbei wiederum bevorzugt sind Ethanol, Diethylphthalat, Propylenglycol, Dipropylenglycol, Triethylcitrat, Benzylbenzoat und Isopropylmyristat.

Eine "Riechstoffzusammensetzung" ist im Sinne der vorliegenden Erfindung eine Mischung, die neben einem "erfindungsgemäß hergestellten Riechstoff" oder einem hierin definierten Stoffgemisch aus mehreren "erfindungsgemäß hergestellten Riechstoffen" gegebenenfalls mindestens einen weiteren Riechstoff umfasst. Insbesondere kann es sich bei einer solchen Riechstoffkomposition um eine Parfümkomposition (ein Parfümöl) handeln.

Erfindungsgemäße Riechstoffkompositionen enthalten, bezogen auf die Gesamtmenge der Riechstoffkomposition, z.B. eine Menge von einem "erfindungsgemäß hergestellten Riechstoff" oder einem hierin definierten Stoffgemisch aus mehreren "erfindungsgemäß hergestellten Riechstoffen" von 0,01 bis 65 Gew.-%, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%. Das Gewichtsverhältnis von einer erfindungsgemäß hergestellter Verbindung/erfindungsgemäß hergestellten Verbindungen zu der Gesamtmenge an weiteren Riechstoffen liegt z.B. im Bereich von 1:1000 bis 1:0,5 , vorzugsweise im Bereich von 1:700 bis 1:1, besonders bevorzugt im Bereich von 1:500 bis 1:10.

Erfindungsgemäße Riechstoffkompositionen enthalten, bezogen auf die Gesamtmenge der Riechstoffkomposition, z.B. eine Menge von "erfindungsgemäß hergestelltem Riechstoff" oder einem hierin definierten Stoffgemisch aus mehreren "erfindungsgemäß hergestellten Riechstoffen" von 0,01 bis 65 Gew.-% und, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%. Das Gewichtsverhältnis von erfindungsgemäß hergestellter Verbindung/erfindungsgemäß hergestellten Verbindungen zu der Gesamtmenge an weiteren (davon verschiedenen) Riechstoffen liegt z.B. im Bereich von 1:1000 bis 1:0,5 , vorzugsweise im Bereich von 1:700 bis 1:1, besonders bevorzugt im Bereich von 1:500 bis 1:10.

Obige Angaben zum Gehalt an erfindungsgemäß hergestellten Verbindungen gelten sinngemäß auch für die bevorzugten Verbindungen der Formeln **I, II, III** und **IV,** insbesondere auch für Stoffgemische der Verbindungen der Formeln **I, II, III** und **IV.**

### Weitere Riechstoffe:

Erfindungsgemäße Riechstoffkompositionen enthalten neben dem "erfindungsgemäß hergestelltem Riechstoff" oder einem hierin definierten Stoffgemisch aus mehreren "erfindungsgemäß hergestellten Riechstoffen" zumindest einen weiteren Riechstoff, vorzugsweise 2, 3, 4, 5, 6, 7, 8 oder mehr weitere Riechstoffe, wobei weitere Riechstoffe z.B. ausgewählt sind unter:
Alpha-Hexylzimtaldehyd, 2-Phenoxyethylisobutyrat (Phenirat¹), Dihydromyrcenol (2,6-Dimethyl-7-octen-2-ol), Methyldihydrojasmonat (vorzugsweise mit einem Gehalt an cis-Isomerem von mehr als 60 Gew.-%) (Hedione⁹, Hedione HC⁹), 4,6,6,7,8,8-Hexamethyl-1,3,4,6,7,8-hexahydrocyclopenta[g]benzopyran (Galaxolid³), Tetrahydrolinalool (3,7-Dimethyloctan-3-ol), Ethyllinalool, Benzylsalicylat, 2-Methyl-3-(4-*tert*-butylphenyl)propanal (Lilial²), Zimtalkohol, 4,7-Methano-3a,4,5,6,7,7a-hexahydro-5-indenylacetat und/oder 4,7-Methano-3a,4,5,6,7,7a-hexahydro-6-indenylacetat (Herbaflorat¹), Citronellol, Citronellylacetat, Tetrahydrogeraniol, Vanillin, Linalylacetat, Styrolylacetat (1-Phenylethylacetat), Octahydro-2,3,8,8-tetramethyl-2-acetonaphthon und/oder 2-Acetyl-1,2,3,4,6,7,8-octahydro-2,3,8,8-tetramethyl-naphtalin (Iso E Super³), Hexylsalicylat, 4-*tert*-Butylcyclohexylacetat (Oryclone¹), 2-*tert*-Butylcyclohexylacetat (Agrumex HC¹), Alphalonon (4-(2,2,6-Trimethyl-2-cyclohexen-1-yl)-3-buten-2-on), n-alpha-Methylionon, alpha-Isomethylionon, Coumarin, Terpinylacetat, 2-Phenylethylalkohol, 4-(4-Hydroxy-4-methylpentyl)-3-cyclohexencarboxaldehyd (Lyral³), alpha-Amylzimtaldehyd, Ethylenbrassylat, (E)- und/oder (Z)-3-Methylcyclopentadec-5-enon (Muscenon⁹), 15-Pentadec-11-enolid und/oder 15-Pentadec-12-enolid (Globalide¹), 15-Cyclopentadecanolid (Macrolide¹), 1-(5,6,7,8-Tetrahydro-3,5,5,6,8,8-hexamethyl-2-naphthalenyl)ethanon (Tonalid¹⁰), 2-Isobutyl-4-methyltetrahydro-2H-pyran-4-ol (Florol⁹), 2-Ethyl-4-(2,2,3-trimethyl-3-cyclopenten-1-yl)-2-buten-1-ol (Sandolen¹), *cis*-3-Hexenylacetat, *trans*-3-Hexenylacetat, *trans*-2,-*cis*-6-Nonadienol, 2,4-Dimethyl-3-cyclohexencarboxaldehyd (Vertocitral¹), 2,4,4,7-Tetramethyl-oct-6-en-3-on (Claritone¹), 2,6-Dimethyl-5-hepten-1-al (Melonal²), Borneol, 3-(3-Isopropylphenyl)butanal (Florhydral²), 2-Methyl-3-(3,4-methylendioxyphenyl)propanal (Helional³), 3-(4-Ethylphenyl)-2,2-dimethylpropanal (Florazon¹), 7-Methyl-2H-1,5-benzodioxepin-3(4H)-on (Calone1951⁵), 3,3,5-Trimethylcyclohexylacetat (vorzugsweise mit einem Gehalt an cis-Isomeren von 70 Gew.-%) oder mehr und 2,5,5-Trimethyl-1,2,3,4,4a,5,6,7-octahydronaphthalin-2-ol (Ambrinol S¹). Die vorangehend genannten Riechstoffe werden im Rahmen der vorliegenden Erfindung demnach bevorzugt mit erfindungsgemäßen Mischungen kombiniert.

Sofern vorstehend Handelsnamen angegeben sind, beziehen sich diese auf folgende Quellen:
¹Handelsname Symrise GmbH, Deutschland;
²Handelsname Givaudan AG, Schweiz;
³Handelsname International Flavors & Fragrances Inc., USA;
⁵Handelsname Danisco Seillans S.A., Frankreich;
⁹Handelsname Firmenich S.A., Schweiz;
¹⁰Handelsname PFW Aroma Chemicals B.V., Niederlande.

Weitere Riechstoffe, mit denen (E/Z)-Cyclopentadec-7/8-en-1-on z.B. zu einer Riechstoffkomposition kombiniert werden können, finden sich z.B. in S. Arctander, Perfume and Flavor Chemicals, Vol. I und II, Montclair, N. J., 1969, Selbstverlag oder K. Bauer, D. Garbe und H. Surburg, Common Fragrance and Flavor Materials, 4rd. Ed., Wiley- VCH, Weinheim 2001. Im Einzelnen seien genannt:
Extrakte aus natürlichen Rohstoffen wie etherischen Ölen, Concretes, Absolues, Resine, Resinoide, Balsame, Tinkturen wie z.B.
Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copalvabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptuscitriodora Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzelriechstoffe aus der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methylenheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyl-oxyacetaldehyd; (E/Z)-1-(1-Methoxy-propoxy)--3-hexen; der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; (E/Z)-Ethyl2,4-decadienoat; Methyl-2-octinat; Methyl-2-noninat; Allyl-2-isoamyloxyacetat; Methyl-3,7-dimethyl-2,6-octadienoat; 4-Methyl-2-pentylcrotonat;
der acyclischen Terpenalkohole wie z.B. Geraniol; Nerol; Linalool; Lavadulol; Nerolidol; Farnesol; Tetrahydrolinalool; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,1 -Trimethyl-9-undecenal; Geranylaceton; sowie die Dimethyl- und Diethylacetale von Geranial, Neral, 7-Hydroxy-3,7-dimethyloctanal; der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-*tert*-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha-3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-rimethyl-3-cyclopent-1-yl)-2-buten-1-ol; 2-Ethyl-4-(2,2,3-Trimethyl-3-cyclopent-1-yl)-2-buten-1-ol; 3-Methyl-5-(2,2,3-Trimethyl-3-cyclopent-1-yl)-pentan-2-ol; 3-Methyl-5-(2,2,3-Trimethyl-3-cyclopent-1-yl)-4-penten-2-ol; 3,3-Dimethyl-5-(2,2,3-Trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-*tert*-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; *cis*-3-Methylpent-2-en-1-yl-cyclopent-2-en-1-on; 3-Methyl-2-pentyl-2-cyclopenten-1-on; 3-Methyl-4-cyclopenta-decenon; 3-Methyl-5-cyclopentadecenon; 3-Methylcyclopentadecanon; 4-(1-Ethoxyvinyl)-3,3,5,5-tetramethylcyclohexanon; 4-*tert*-Pentylcyclohexanon; Cyclohexadec-5-en-1-on; 6,7-Dihydro-1,1,2,3,3-pentamethyl-4(5H)-indanon; 8-Cyclohexadecen-1-on; 7-Cyclohexadecen-1-on; (7/8)-Cyclohexadecen-1-on; 9-Cyclohepta-decen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-Trimethylcyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z.B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; *tert*-Butyl-(2,4-dimethyl-3-cyclohexen-1-yl)keton;
der Ester cyclischer Alkohole wie z.B. 2-*tert*-Butylcyclohexylacetat; 4-*tert*-Butylcyclohexylacetat; 2-*tert*-Pentylcyclohexylacetat; 4-*tert*-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5 bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z.B. Allyl-3-cyclohexylpropionat; Allylcyclohexyloxyacetat; *cis-* und *trans*-Methyldihydrojasmonat; *cis-* und *trans*-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol, 2-Phenylethylalkohol, 3-Phenylpropanol; 2-Phenylpropanol; 2-Phenoxyethanol; 2,2-Dimethyl-3-phenylpropanol; 2,2-Dimethyl-3-(3-methylphenyl)-propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)-ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-*tert-*butylphenyl)propanal; 2-Methyl-3-(4-isobutylphenyl)propanal; 3-(4-*tert*-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4 Methylacetophenon; 4-Methoxyacetophenon; 4-*tert*-Butyl-2,6-dimethylacetophenon; 4-Phenyl-2-butanon; 4-(4-Hydroxyphenyl)-2-butanon; 1-(2-Naphthalenyl)ethanon; 2-Benzofuranylethanon;(3-Methyl-2-benzofuranyl)ethanon; Benzophenon; 1,1,2,3,3,6-Hexamethyl-5-indanylmethylketon; *6-tert-*Butyl-1,1-dimethyl-4-indanylmethylketon; 1-[2,3-Dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; *cis*-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-*tert*-butylacetophenon; Zimtsäurenitril; 3-Methyl-5-phenyl-2-pentensäurenitril; 3-Methyl-5-phenylpentansäurenitril; Methylanthranilat; Methyl-*N-*methylanthranilat; Schiff'sche Basen von Methylanthranilat mit 7-Hydroxy-3,7-dimethyloctanal, 2-Methyl-3-(4-*tert*-butylphenyl)propanal oder 2,4-Dimethyl-3-cyclohexencarbaldehyd; 6-Isopropylchinolin; 6-Isobutylchinolin; 6-*sec*-Butylchinolin; 2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; *cis-* und *trans*-11-Pentadecen-1,15-olid; *cis-* und *trans*-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

### c2) Riechstoffhaltige Artikel

Erfindungsgemäß herstellte Riechstoffe oder erfindungsgemäße Riechstoffkompositionen können in eine Reihe von Produkten eingearbeitet bzw. auf solche Produkte appliziert werden.

Erfindungsgemäße Riechstoffe können bei der Herstellung parfümierter Artikel eingesetzt. Die olfaktorischen Eigenschaften ebenso wie die stofflichen Eigenschaften (wie Löslichkeit in gängigen Lösungsmitteln und Kompatibilität mit gängigen weiteren Bestandteilen derartiger Produkte) sowie die toxikologische Unbedenklichkeit der erfindungsgemäßen Riechstoffe unterstreichen ihre besondere Eignung für die genannten Einsatzzwecke. Die positiven Eigenschaften tragen dazu bei, dass die erfindungsgemäß verwendeten Riechstoffe und die erfindungsgemäßen Riechstoffkompositionen besonders bevorzugt in Parfümerzeugnissen, Körperpflegeprodukten, Hygieneartikeln, Textilwaschmittel sowie in Reinigungsmitteln für feste Oberflächen eingesetzt werden.

Der parfümierte Artikel ist z.B. ausgewählt unter Parfümerzeugnissen, Körperpflegeprodukten, Hygieneartikeln, Textilwaschmitteln und Reinigungsmitteln für feste Oberflächen. Bevorzugte erfindungsgemäße parfümierte Artikel sind weiterhin ausgewählt unter:
Parfümerzeugnissen, ausgewählt unter Parfüm-Extrakten, Eau de Parfums, Eau de Toilettes, Eau de Colognes, Eau de Solide, Extrait Parfum, Luftverbesserern in flüssiger, gelartiger oder auf einem festen Träger aufgebrachter Form, Aerosolsprays, Duftreinigern und -ölen;
Körperpflegeprodukten, ausgewählt unter Rasierwässern, Pre-shave-Produkten, Splash-Colognes, festen und flüssigen Seifen, Duschgelen, Shampoos, Rasierseifen, Rasierschäumen, Badeölen, kosmetischen Emulsionen vom Öl-in-Wasser-, vom Wasser-in-ÖI- und vom Wasser-in-ÖI-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und - lotionen, Bräunungscremes und -lotionen, Haarpflegeprodukten wie z.B. Haarsprays, Haargelen, festigende Haarlotionen, Haarspülungen, Haarshampoo, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara, Zahnpasta, Zahnseide;
Hygieneartikeln, ausgewählt unter Kerzen, Lampenölen, Räucherstäbchen, Insektiziden, Repellentien, Treibstoffen, Rostentfernern, parfümierten Erfrischungstüchern, Achselpads, Babywindeln, Damenbinden, Toilettenpapier, Kosmetiktüchern, Taschentüchern, Spülmaschinendeo;
Reinigungsmitteln für feste Oberflächen, ausgewählt unter parfümierten sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Wachsen und Polituren wie Möbelpolituren, Fußbodenwachsen, Schuhcremes, Desinfektionsmitteln, Oberflächendesinfektionsmitteln und Sanitärreinigern, Bremsenreinigern, Rohrreinigern, Entkalkern, Grill- und Backofenreinigern, Algen- und Moosentfernern, Schimmelentfernern, Fassadenreinigungsmitteln;
Textilwaschmitteln, ausgewählt unter flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten.

Einem weiteren Aspekt zufolge sind die erfindungsgemäß verwendeten Riechstoffe und die erfindungsgemäßen Riechstoffkompositionen für den Einsatz in tensidhaltigen parfümierten Artikeln geeignet. Gesucht werden nämlich - insbesondere für die Parfümierung von tensidhaltigen Formulierungen wie zum Beispiel Reinigungsmitteln (insbesondere Geschirrspülmittel und Allzweckreiniger) - häufig Riechstoffe und/oder Riechstoffkompositionen mit einer Rosenkopfnote und ausgeprägter Natürlichkeit.

Einem weiteren Aspekt zufolge können erfindungsgemäß verwendete Riechstoffe und erfindungsgemäße Riechstoffkompositionen als Mittel zum Versehen von (a) Haaren oder (b) textilen Fasern mit der Geruchsnote rosig verwendet werden.

Die erfindungsgemäß zu verwendenden Riechstoffe und erfindungsgemäße Riechstoffkompositionen eignen sich daher besonders gut für den Einsatz in tensidhaltigen parfümierten Artikeln.

Bevorzugt ist es, wenn der parfümierte Artikel einer der folgenden ist:
- ein saures, alkalisches oder neutrales Reinigungsmittel, das insbesondere aus der Gruppe ausgewählt ist bestehend aus Allzweckreinigern, Fußbodenreinigern, Fensterglasreinigern, Geschirrspülmitteln, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektions-mitteln, Oberflächendesinfektionsmitteln,
- ein Luftverbesserer in flüssiger, gelartiger oder auf einem festen Träger aufgebrachter Form oder als Aerosolspray,
- ein Wachs oder eine Politur, die insbesondere aus der Gruppe ausgewählt ist bestehend aus Möbelpolituren, Fußbodenwachsen und Schuhcremes, oder
- ein Körperpflegemittel, das insbesondere aus der Gruppe ausgewählt ist bestehend aus Duschgelen und Shampoos Rasierseifen, Rasierschäumen, Badeölen, kosmetischen Emulsionen vom Öl-in-Wasser-, vom Wasser-in-ÖI- und vom Wasser-in-ÖI-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Rollons, Deosticks, Deocremes, Produkten der dekorativen Kosmetik.

Inhaltstoffe, mit denen erfindungsgemäß verwendete Riechstoffe oder erfindungsgemäße Riechstoffkompositionen vorzugsweise kombiniert werden können, sind beispielsweise: Konservierungsmittel, Abrasiva, Antiaknemittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, Schweiss hemmende Mittel, antiseptische Mittel, Antistatika, Binder, Puffer, Trägermaterialien, Chelatbildner, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, a-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Einem weiteren Aspekt zufolge verwendet man die Riechstoffe bei der Herstellung der parfümierten Artikel in flüssiger Form, unverdünnt oder mit einem Lösungsmittel verdünnt oder in Form einer Riechstoffkomposition. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw. Für die genannten Lösungsmittel gilt, dass diese, im Falle des Vorhandenseins eigener olfaktorischer Eigenschaften, ausschließlich dem Bestandteil "Lösungsmittel" und nicht den "Riechstoffen" zuzuordnen sind.

Die in den erfindungsgemäßen parfümierten Artikeln enthaltenen Riechstoffe und/oder Riechstoffkompositionen können dabei in einer Ausführungsform an einem Trägerstoff absorbiert sein, der sowohl für eine feine Verteilung des Riechstoffs oder der Riechstoffkomposition im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die erfindungsgemäß verwendeten Riechstoffe und die erfindungsgemäßen Riechstoffkompositionen können auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden. Die Eigenschaften können durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung kann beispielsweise durch das sogenannte Koazervationsverfahren mit Hilfe von Kapselmaterialien, z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschlusskomplexe können z.B. durch Eintragen von Dispersionen von Riechstoffkompositionen und Cyclodextrinen oder Harnstoffderivaten in ein geeignetes Lösungsmittel, z.B. Wasser, hergestellt werden. Extrusionsprodukte können durch Verschmelzen erfindungsgemäß verwendeter Riechstoffe und erfindungsgemäßer Riechstoffkompositionen mit einem geeigneten wachsartigen Stoff und durch Extrusion mit nachfolgender Erstarrung, ggf. in einem geeigneten Lösungsmittel, z.B. Isopropanol, hergestellt werden.

### c3) Herstellung erfindungsgemäßer Riechstoffe

### i) N₂O-Oxidation gemäß Stufe i)

Ausgangsverbindung (die sowohl in stereoisomerenreiner Form als auch in Form von Stereoisomeren-Gemischen eingesetzt werden kann) ist das cyclisches Olefin Cyclohexadeca-1,9-dien, das entweder käuflich erhältlich ist oder gemäß Beispiel 2 der WO 2012/084673 hergestellt werden kann.

Insbesondere wird dabei ein cyclisches Olefin durch Umsetzung mit Distickstoffmonoxid oxidiert. Distickstoffmonoxid kann dabei rein oder ggf. im Gemisch mit anderen bei Reaktionsbedingungen gasförmigen Substanzen, wie z.B. Kohlenstoffdioxid zur Verdünnung eingesetzt werden.

Dabei kann für die Umsetzung des cyclischen Olefins mit Distickstoffmonoxid in Substanz oder in Gegenwart mindestens eines geeigneten Lösungsmittels oder Verdünnungsmittels erfolgen. Vorzugsweise erfolgt die Umsetzung in Substanz. Im Wesentlichen alle gängigen Lösungs- und/oder Verdünnungsmittel sind hierbei geeignet, jedoch unter der Maßgabe, dass sie weder eine C-C-Doppelbindung, noch eine C-C-Dreifachbindung, noch eine Aldehydgruppe aufweisen. Als geeignete Lösungsmittel sind unter anderem zu nennen: cyclische Alkane, beispielsweise Cyclohexan, Cyclopentan, Cyclooctan, Cyclododecan oder gesättigte aliphatische oder aromatische, gegebenenfalls alkylsubstituierte Kohlenwasserstoffe zu nennen.

Die Temperatur bei der Umsetzung liegt z.B. bei 140 bis 350°C, wie insbesondere bei 180 bis 320°C oder bei 200 bis 300 °C. Es ist auch möglich, die Umsetzung bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden. Insbesondere ist die Umsetzungstemperatur aber im Wesentlichen konstant. Die Reaktion kann aber auch vorzugsweise adiabatisch durchgeführt werden, so dass im Reaktor die Temperatur steigt.

Der Druck bei der Umsetzung des cyclischen Olefins mit Distickstoffmonoxid liegt insbesondere höher als der Eigendruck des Edukt bzw. Produktgemisches bei der gewählten Reaktionstemperatur oder den gewählten Reaktionstemperaturen. Der Druck liegt z.B. bei 1 bis 1000 bar, wie z.B. bei 40 bis 300 bar oder bei 50 bis 200 bar.

Es ist möglich, die Umsetzung des cyclischen Olefins mit Distickstoffmonoxid bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden. Insbesondere ist Druck während der Umsetzung aber im Wesentlichen konstant.

Hinsichtlich der für die Umsetzung (im Labor- oder Produktionsmaßstab) einsetzbaren Reaktoren gibt es keine besonderen Beschränkungen. Insbesondere kann die Umsetzung in Batch-Fahrweise oder in kontinuierlicher Fahrweise erfolgen. Folglich können beispielsweise als Reaktoren mindestens ein CSTR (Continuous Stirred Tank Reactor) mit mindestens einem internen und/oder mindestens einem externen Wärmetauscher, mindestens ein Rohrreaktor, mindestens ein Rohrbündelreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden. Insbesondere wird die Umsetzung mit Distickstoffmonoxid in einem einzigen Reaktor durchgeführt. Beispielsweise kann die Umsetzung in kontinuierlicher Fahrweise oder in Batch-Fahrweise erfolgen.

Die Verweilzeit der Reaktionsmischung im Reaktor liegt im Allgemeinen im Bereich von 0,1 bis 40 Stunden, bevorzugt im Bereich von 1 bis 30 Stunden, weiter bevorzugt im Bereich von 2 bis 25 Stunden.

Im Feed liegt das Molverhältnis von Distickstoffmonoxid und dem cyclischen Olefin im Allgemeinen im Bereich von 0,01 bis 30, wie z.B. im Bereich von 0,03 bis10, besonders bevorzugt im Bereich von 0,05 bis 1 und ganz besonders bevorzugt im Bereich von 0,08 bis 0,2.

Da Distickstoffmonoxid bevorzugt im Unterschuss eingesetzt wird, wird nur ein Teil des Cyclohexadeca-1,9-diens umgesetzt. Nicht umgesetztes Cyclohexadeca-1,9-dien wird destillativ vom Reaktionsprodukt getrennt und der Reaktion wieder zugeführt. Dabei fällt das nicht umgesetzte Cyclohexadeca-1,9-dien als Kopfprodukt und das Reaktionsprodukt als Sumpfprodukt der Kolonne an. Die Destillation findet hierbei z.B. bei 20 mbar Kopfdruck und 210 °C Sumpftemperatur statt. Der Differenzdruck über die Kolonne beträgt etwa 18 mbar. Die Kolonne kann mit einer strukturierten Gewebepackung vom Typ Montz A3 ausgestattet sein. Die Packungshöhe beträgt z.B.4 m und der Zulauf liegt z.B bei 2 m.

Aus dem erhaltenen Sumpfaustrag kann anschließend das als Nebenkomponente gebildete Cyclopentadecenylcarbaldehyd destillativ isoliert (angereichert) werden und kann durch Chromatographie weiter gereinigt werden. Insbesondere wird aber das so erhaltene Rohproduktgemisch aus Cyclohexadec-8-en-1-on und Carbaldehyden direkt in Stufe ii) eingesetzt.

Bevorzugt erfolgt somit keine weitere Aufreinigung, sondern die direkte Umsetzung des Reaktionsgemischs (RG1) in Stufe ii).

In einer weiteren bevorzugten Variante erfolgt eine weitere Reinigung mittels Destillation zum Erhalt des Reaktionsgemisches RG1a.

Im Falle einer zusätzlichen Reinigung kann diese z.B. destillativ (wie insbesondere durch fraktionierte Destillation, vorzugsweise bei vermindertem Druck) oder chromatographisch erfolgen, bevorzugt erfolgt die Reinigung mittels Destillation. Geeignete Reinigungsmethoden sind dem Fachmann geläufig. Die Reinigung kann z.B. batchwiese als auch kontinuierlich erfolgen.

Beispielsweise kann die Destillation mittels Destillationskolonne mit dem Fachmann bekannten Packungen verwendet. Die optimalen Destillationsbedingungen sind vom Fachmann ohne unzumutbaren Aufwand festlegbar. Die Destillation kann insbesondere im Vakuum, beispielsweise bei einem Druck < 1000 mbar, < 500 mbar, < 300 mbar, < 100 mbar oder < 10 mbar durchgeführt werden. Die verwendete Destillationskolonne kann mehrere, wie z.B. mindestens 20, mindestens 25 oder mindestens 30 theoretische, wie z.B. bis zu 70 Trennstufen aufweisen. Das Rücklaufverhältnis kann z.B. im Bereich von etwa 5 bis 100 liegen und mindestens 20, mindestens 25 oder mindestens 30 betragen und beträgt insbesondere etwa 100 für eine besonders vorteilhafte Fraktionierung.

Beispielsweise kann auch eine Säulenchromatographie an Stelle oder im Anschluss an eine destillative Reinigung erfolgen. Hierzu verwendet man dem Fachmann bekannte Säulenmaterialen und Laufmittel. Die optimalen Chromatographiebedingungen, wie Säulengeometrie und Geschwindigkeit des Laufmittels, sind vom Fachmann ohne unzumutbaren Aufwand festlegbar.

Beispiele für geeignete Säulenmaterialien sind polare Adsorptionsmittel wie z. B. Eisenoxid Fe₂O₃, Aluminiumoxid, Kohlenhydrate oder Kieselgel mit oder ohne Zusätze wie z. B. Fluoreszenzindikatoren oder Gips.

Beispiele für geeignete Laufmittel sind: aliphatische oder aromatische Laufmittel, wie z.B. Alkane oder Cycloalkane, wie z.B. Pentan, Petrolether, Hexan, Heptan, Toluol oder die korrespondierenden cyclischen Verbindungen; aliphatische Ether, Ester oder, wie z.B. Et₂O, MTBE, EtOAc, Aceton oder Mischungen solcher Laufmitteln, wie z. B. Hexan/MTBE, Hexan/EtOAc, Pentan/Et₂O, Petrolether/Et₂O.

Man kann dabei eine oder mehrere Fraktionen (F2) mit erhöhtem Gehalt an Carbaldehyd mit einem Carbaldehydgehalt von mehr als 20, wie z.B. mehr als 30, mehr als 40, mehr als 50, mehr als 60, mehr als 70 oder mehr als 80 %. isolieren. Die %-Angaben betreffen jeweils die durch GC-Analyse ermittelten Flächen-% und entsprechen in etwa den Gew.-% Anteilen, bezogen auf den Feststoffgehalt der untersuchten Mischung.

Weiterhin kann man eine oder mehrere Fraktionen (F1) isolieren, angereichert an Cyclohexadec-8-en-1-on (Gehalt etwa 98%), und insbesondere mit weniger als 1% Cyclopentadecenylcarbaldehyde, Die %-Angaben betreffen jeweils die durch GC-Analyse ermittelten Flächen-% und entsprechen in etwa den Gew.-% Anteilen, bezogen auf den Feststoffgehalt der untersuchten Mischung.

Der Carbaldehyde und Cyclohexadec-8-en-1-on können dabei in stereoisomerenreiner Form, oder insbesondere als Gemisch zweier oder mehrerer Stereoisomeren, isoliert werden.

### ii) Oxidative Decarbonylierung gemäß Stufe ii)

Ausgehend vom Reaktionsprodukt der Stufe i) (d.h. RG1, RG1a oder F2) erfolgt die oxidative Decarbonylierung in Anlehnung an die aus dem Stand der Technik bekannten Cu-(II)- basierten Decarbonylierungen vgl. z.B. a) Tetrahedron Letters 1969, 12, 985; US3496197; b) Tetrahedron Letters 1995, 4641, c) Org. Lett. 2010, 2630, d) Bioorg. Med. Chem. Lett. 2013, 23, 5949, e) Chin. Chem. Lett. 2014, 25, 771.)

Erfindungsgemäß erfolgt die katalytische Umsetzung des cyclischen Carbaldehyden mit dem molekularen Sauerstoff in Gegenwart mindestens eines geeigneten Lösungsmittels oder Verdünnungsmittels. Die Reaktion kann auch ohne Lösungsmittel durchgeführt werden.

Sauerstoff kann dabei als reiner Sauerstoff oder vorzugsweise als Bestandteil von Umgebungsluft oder Magerluft im Prozess verwendet werden. Beispielsweise kann die Reaktion mit Luftströmen im Bereich von 0,5-100 Nl/h, bevorzugt etwa 50 Nl/h durchgeführt. Die Luft kann beispilesweise mittels einer Fritte in die Reaktionsmischung geleitet werden. Alternativ kann man das Gas auch mittels einem Rohr oder einer Düse in die Reaktion leiten.

Als geeignete Lösungsmittel sind unter anderem zu nennen: polare, aprotische Lösungsmittel wie Dimethylformamid (DMF), Hexamethylphosphoramid (HMPA), Dimethylsulfoxid (DMSO), Tetramethylharnstoff und Dimethylacetamid, sowie Mischungen davon. Weitere geeignete organische Lösungsmittel, welche alleine oder in Kombination mit obigen aprotischen organischen Lösungsmitteln einsetzbar sind, sind Alkanole, wie beispielsweise Methanol, Ethanol, Propanol, Isopropanol oder Butanole, wie *tert*-Butanol sowie Tetrahydrofuran, Dioxan oder Benzol.

As Katalysator verwendet man Cu-(II)- basierte, insbesondere homogene Katalysatoren. Diese werden bevorzugt in situ im Reaktionsansatz gebildet, indem man zu einem Cu-(II)-Salz einen insbesondere zweizähnigen Liganden, vorzugsweise Diamin-Liganden, gibt.

Das erfindungsgemäß eingesetzte Cu-(II)-Salz ist beispielsweise ausgewählt unter Cu-(II)-acetat, -formiat, -sulfat, -chlorid oder-nitrat, Vorzugsweise wird Cu(OAc)₂ eingesetzt.

Geeignete Komplexliganden sind insbesondere zweizähnige Kupfer komplexierende Amin-Liganden, wie *N,N,N',N'*-Tetramethylethylendiamin (TMEDA), 1,10-Phenantrolin und 2,2'-Bipyridin. Vorzugsweise wird TMEDA eingesetzt.

Komplexligand und Cu-(II)-Salz werden in etwa äquimolarem Verhältnis eingesetzt. Der molare Anteil an Komplex liegt bei etwa 0,1 bis 10, insbesondre 1 bis 5, vorzugsweise etwa 2,5 mol-%, bezogen auf den eingesetzten Carbaldehyd.

Insbesondere wird die Decarbonylierung zusätzlich in Gegenwart einer organischen Base erfolgt. Die im erfindungsgemäßen Verfahren beispielsweise eingesetzte Base ist ausgewählt unter Diazabicycloalkanen, wie z. B. Diazabicyclooctan (DABCO), Diazabicycloundecen (DBU), Diazobicyclononan (DBN), tertiären Aminen wie Trimethylamin, Triethylamin, Diisopropylethylamin oder Tripropylamin, N,N-Dimethylpiperazin, N-Methylpyridin, N-Methylpyrrolidon, Quinuclidin und dergleichen. Vorzugsweise wird DBU eingesetzt. Die Base wird dabei in einem Anteil von 0,1 - 1 equiv, 0,2 - 0,8 equiv oder besonders bevorzugt 0,4 - 0,6 equiv bezogen auf den eingesetzten Carbaldehyden eingesetzt.

Die Temperatur bei der Umsetzung liegt, je nach verwendeten Reaktanden und Lösungsmittel z.B. bei 20 bis 100°C, wie insbesondere bei 30 bis 80 °C insbesondere bei 40 bis 60°C. Es ist auch möglich, die Umsetzung bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden. Insbesondere ist die Umsetzungstemperatur aber im Wesentlichen konstant.

Der Druck bei der Umsetzung des Carbaldehyden mit Sauerstoff liegt insbesondere bei Umgebungsdruck oder etwa im Bereich des Eigendrucks des Edukt bzw. Produktgemisches bei der gewählten Reaktionstemperatur oder den gewählten Reaktionstemperaturen. Der Druck liegt z.B. bei 1 bis 5 bar, wie z.B. bei 1 bis 3 bar vorzugsweise etwa 1 bar.

Es ist möglich, die Umsetzung bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden. Insbesondere ist Druck während der Umsetzung aber im Wesentlichen konstant.

Insbesondere kann die Umsetzung in Batch-Fahrweise oder in kontinuierlicher Fahrweise erfolgen.

Hinsichtlich der für die Umsetzung (im Labor- oder Produktionsmaßstab) einsetzbaren Reaktionsgefäße gibt es keine besonderen Beschränkungen. Im Falle einer Verwendung eines Reaktors können beispielsweise übliche Rührreaktoren, CSTR (Continuous Stirred Tank Reactor), jeweils mit oder ohne internen und/oder externen Wärmetauscher, ein Rohrreaktor, ein Rohrbündelreaktor oder ein Schlaufenreaktor eingesetzt werden. Ebenso ist es möglich, den Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden. Insbesondere wird die Umsetzung jedoch in einem einzigen Reaktor, insbesondere Rührreaktor, durchgeführt.

Die Verweilzeit der Reaktionsmischung im Reaktor liegt im Allgemeinen im Bereich von 0,1 bis 40 Stunden, bevorzugt im Bereich von 5 bis 30 Stunden, weiter bevorzugt im Bereich von 2 bis 7 Stunden.

Insbesondere kann die Umsetzung wie folgt durchgeführt werden:
Ein Reaktionsgemisch (RG1, RG1a) bestehend aus einem Überschuss an (E/Z)-Cyclohexadec-8-en-1-on und einer im Unterschuss enthaltenen Menge an Cyclopentadec-7/8-enylcarbaldehyd, oder ein Reaktionsgemisch (F2) bestehend aus einem Überschuss an Cyclopentadec-7/8-enylcarbaldehyd und einer im Unterschuss enthaltenen Menge an (E/Z)-Cyclohexadec-8-en-1-on wird in DMF gelöst. Zu der Mischung gibt man als Base Diazabicycloundecen (DBU, 0,5 bis 1,5, z.B. 0.6 eq bezogen auf Carbaldehyd) hinzugegeben. Durch die Mischung wurde kontinuierlich ein Luftstrom geleitet. Der zweizähnige Komplex-Ligand TMEDA (1 bis 5 mol.-%, wie z.B. 2 mol% bezogen auf Carbaldehyd) und Cu(OAc)₂ (1 bis 5 mol.-%, 2 mol%) werden in DMF gelöst. Die Cu-TMEDA-Mischung wird sodann kontinuierlich der Reaktionslösung hinzugetropft oder direkt (in einer Portion) zugegeben. Die Reaktion wurde bei 40 bis 60, wie z.B. 50°C für 3 bis 30 h, wie z.B. 5 h gerührt. Anschließend wird EtOAc und Wasser hinzugegeben. Die wässrige Phase wird mittels 98%iger H₂SO₄ auf pH 4 eingestellt. Die organische Phase wird extrahiert. Anschließend wird die wässrige Phase gegeben falls nochmals mit EtOAc gewaschen. Die vereinten organischen Phasen werden getrocknet, filtriert und im Vakuum eingeengt.

Der Rückstand wird dann ggf. weiter aufgebarbeitet. Beispielsweise führt man eine fraktionierte Destillation durch. Die erfindungsgemäßen Cyclopentadecenone können dadurch von nicht umgesetzten Cyclohexadec-8-en-1-on getrennt werden. Die Cyclopentadecenone können im Anschluss gegebenenfalls von Leichtsiedern, welche durch Destillation nicht abgetrennt wurden, säulenchromatographisch getrennt werden. Hierzu eignet sich z.B. Kieselgel als stationäre Phase und als Laufmittel eine Mischung Cyclohexan: EtOAc, wie z.B. durch Elution mittels eines Stufengradienten 100 : 1 / 30 :1 / 20 : 1. Das Produkt wird bei etwa 80:1 eluiert.

Alternativ kann auch anstelle der Fraktionierten Destillation eine Säulenhromatographie erfolgen. Das Gemisch bestehend aus Cyclopentadecenonen und Cyclohexadecenonen kann dabei säulenchromatographisch mit einem Laufmittel Cyclohexan : EtOAc (30 : 1 → 20 : 1) gereinigt. (Säulenmaterial: Kieselgel F₂₅₄)

Die Erfindung wird nun unter Bezugnahme auf folgende nichtlimitierende Ausführungsbeispiele näher erläutert:

### Experimenteller Teil

### Methoden:

### Gaschromatographie (GC)

Trennsäule : CP-Wax 52CB 25m x 0,32mm x1,2µm 1ml/min N₂
Bedingungen: 90°-5min-10°/min-240°-30min Inj/Det 200°/250° (Methode A)
Bedingungen: 80°-3°/min-250°- Inj/Det 200°/250° (Methode B) (Nur Beispiel 2)
Probenvolumen: 0,2 ml

### GC/MS

Trennsäule: CP-Wax 52 CB (1.2 µm Filmdicke), Splitverhältnis 10:1
Bedingungen: 80°-3min-240°-30min 0.2µl
MS-Bedingungen: 25-785 amu, 70 eV

### GC/IR

Detektor: MCT/AWellenlänge 650 - 4000 cm⁻¹
Zellen-/Transfertemperatur 250°C
Scan 6
Resolution 8

### Säu lenchromatoqraphie

Verwendet wurde eine Glassäule mit Frittenboden. Die Säule wurde zu 2/3 gepackt mit aufgeschlemmten Kieselgel F₂₅₄. Das Lösungsmittelgemisch wurde mit einem Überdruck von 0.2-0.4 bar durch die Säule gedrückt.

### Beispiel 1: Umsetzung von Cyclohexadeca-1,9-dien (CHDD) mit N₂O

In einem adiabaten Rohrreaktor (12 m Länge, 10 cm Durchmesser) wurden 2000 g/h CHDD mit 40 g/h N₂O bei einer Reaktoreingangstemperatur von 210°C und 100 bar umgesetzt. Der Umsatz an CHDD betrug 12%. Nicht umgesetztes CHDD wurde mittels Destillation (20 mbar, 190°C Sumpftemperatur) vom Produktgemisch umfassend Globanon®, Cyclopentadecenylcarbaldehyden **I-IV** getrennt und dem System zurückgeführt.

Im Übrigen erfolgte die Reaktion, wie z.B. beschrieben in der PCT/EP2015/072544 oder WO 2012/084673 der Anmelderin.

### Vergleichsbeispiel 1: Destillation des Austrags aus Beispiel 1

Der Reaktionsaustrag aus Beispiel 1 wurde unter Vakuum (< 10 mbar) und einer Sumpftemperatur von 200 °C in einer Kolonne mit einer Trennstufenzahl von 70 und einem Rücklaufverhältnis von 100 fraktioniert destilliert. Das so hergestellte Cyclohexadec-8-enon enthält noch unerwünschte Cyclopentadecenylcarbaldehyde.

Das Destillat wurde olfaktorisch bewertet: Kunststoffnote, aldehydisch, moschus

### Beispiel 2: Cu-katalysierte oxidative Decarbonylierung des Produkts aus Beispiel 1

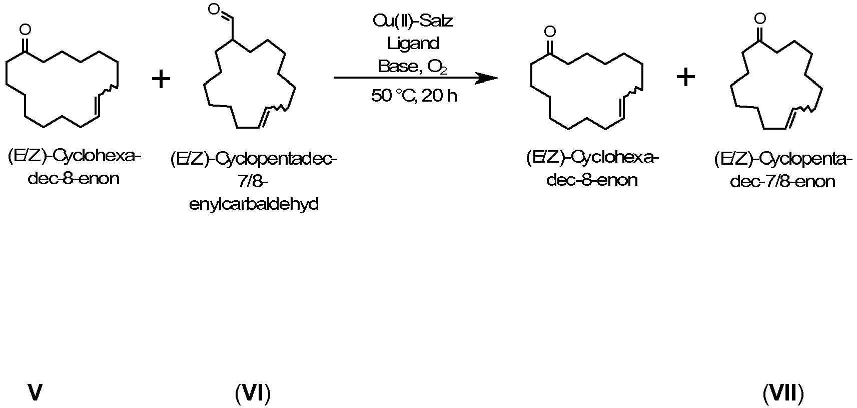

100 g einer Mischung bestehend aus 81% (E/Z)-Cyclohexadec-8-enon (V) und 5.9% Cyclopentadec-7/8-enylcarbaldehyd (**VI**) wurden in 300 ml DMF gelöst. Zu der Mischung wurden 2.3 g Diazabicycloundecen (DBU, 15.2 mmol, 0.6 eq) hinzugegeben. Durch die Mischung wurde kontinuierlich ein Luftstrom geleitet. TMEDA (120 mg, 0.5 mmol, 2 mol%) und Cu(OAc)₂ (90 mg, 0.5 mmol, 2 mol%) wurden in 30 ml DMF gelöst. Die Cu-TMEDA-Mischung wurde über eine Spritzenpumpe kontinuierlich über 8 h der Reaktionslösung hinzugetropft. Die Reaktion wurde bei 50 °C für 20 h gerührt. Anschließend wurden 300 ml EtOAc hinzugegeben sowie 200 ml 0.1 M HCl. Die org. Phase wurde extrahiert. Anschließend wurde die wässrige Phase noch einmal mit EtOAc (2 x 100 ml) gewaschen. Die vereinten organischen Phasen würden über Na₂SO₄ getrocknet, abfiltriert und im Vakuum eingeengt. Der Umsatz der Carbaldehyden betrug > 95%. Die Selektivität der Cyclopentadecenone lag bei 95%. Cyclohexadec-8-enon wurde nicht angegriffen.

Der Rückstand wurde mittels fraktionierter Destillation in einer Drehbandkolonne mit einer Stufenzahl von 20 bei 1 mbar Kopfdruck und 125-129 °C Kopf- und 170-180 °C Sumpftemperatur aufgearbeitet. Das Rücklaufverhältnis lag bei 75.

Die Cyclopentadec-7/8-enone wurde von Cyclohexadec-8-enon (Ausbeute 85%) getrennt.

Die Cyclopentadec-7/8-enone wurden im Anschluss von Leichtsiedern, welche in der Destillation nicht abgetrennt werden konnten, säulenchromatographisch getrennt (Kieselgel, Laufmittel Cyclohexan: EtOAc 100 : 1, 30 : 1, 20 : 1), so dass 1 g eines farblosen Öls mit einer Reinheit von 84% (3.7 mmol) erhalten werden konnte.

Anteil:
(E)-Cyclopentadec-8-enon = 54%
(Z)-Cyclopentadec-8-enon = 30%
(E)-Cyclopentadec-7-enon = 0.45%

¹H NMR (500 MHz, CDCl₃, 25 °C): s = 5.3 (m, 2H), 2.5-2.3 (m, 4H), 2.1-1.9 (m, 4H), 1.7-1.55 (m, 4H), 1.45-1.10 (m, 12H). (des Gemischs)

### trans-Cyclopentadec-8-enon I

¹³C-NMR (125 MHz, CDCl₃, 25 °C): σ = 211.98 (C=O), 130.99 (C=C), 41.49 (2xCH₂), 31.80 (2xCH₂), 28.26 (2xCH₂), 28.21 (2xCH₂), 26.84 (2xCH₂), 26.83 (2xCH₂), 22.91 (2xCH₂).

### cis-Cyclopentadec-8-enon III

¹³C-NMR (125 MHz, CDCl₃, 25 °C): σ = 211.93 (C=O), 130.44 (C=C), 41.57 (2xCH₂), 31.80 (2xCH₂), 28.48 (2xCH₂), 27.68 (2xCH₂), 25.74 (2xCH₂), 23.16 (2xCH₂).
MS m/z = 222, 204, 179, 165, 152, 135, 125, 111, 98, 81, 67, 55, 41.

### trans-Cyclopentadec-8-enon I

IR (ATR) u [cm⁻¹] = 3022, 2934, 2864, 1724, 1449, 1356, 1121, 969.

### cis-Cyclopentadec-8-enon III

IR (ATR) u [cm⁻¹] = 3011, 2935, 2866, 1723, 1456, 1354, 716.

Im GC konnte in der Mischung auch *trans*-Cyclopentadec-7-enon **II** mit einem Anteil an 0.45% nachgewiesen werden. Für eine spektroskopische Auswertung der Verbindung war allerdings nicht genügend Material vorhanden. Mittels GC/MS-IR konnte die Verbindung identifiziert werden

### trans-Cyclopentadec-7-enon II

IR (ATR) u [cm⁻¹] = 3020, 2934, 2864, 1723, 1449, 1353, 1121, 969.

Zusätzlich konnte aus einem weiteren Reaktionsansatz mittels GC/MS-IR die Verbindung **IV** identifiziert werden:
*cis*-Cyclopentadec-7-enon **IV**
IR (ATR) u [cm⁻¹] = 3012, 2936, 2866, 1723, 1457, 1353, 714.

Riechtest (Mischung aus I-III, 84%): grün, aldehydisch, harsch, gasig, nach Moschus (84% Cyclopentadec-8-enon)

### Beispiel 3: Feindestillation des Austrags aus Beispiel 1 unter Erhalt eines ersten Schnitts (F1), der aus Cyclohexadec-8-enon mit nur noch wenig Cyclopentadecenylcarbaldehyden und eines zweiten Schnitts (F2) aus Cyclohexadec-8-enon mit hohem Anteil an Cyclopentadecenylcarbaldehyden

2600 g eines Gemisches aus Beispiel 1 (Rohaustrag aus Oxidation von Cyclohexadeca-1,9-dien mit N₂O) mit in Summe ca. 5% Cyclopentadecenylcarbaldehyd wurden in einer Batchkolonne (Sulzer-Gewebepackung DX, trennwirksame Höhe 2000 mm, Durchmesser 43 mm, Kopfdruck: 5 mbar, Druckverlust über Kolonne: 5 mbar, Sumpftemperatur: 180°C, Sambayverdampfer, Rücklaufverhältnis: 100) fraktioniert destilliert. Gemische aus verschiedenen Anteilen von Cyclopentadec-7/8-enylcarbaldehyd und Cyclohexadec-8-enon konnte dabei in verschiedenen Fraktionen erhalten werden. Der jeweilige Gehalt (Flächen-%; entsprechend in etwa Gew.-%) wurde gaschromatographisch bestimmt.

Insbesondere wurden erhalten:
Schnitt F1: Anteil Cyclohexadec-8-enon Anteil 98%; Anteil Cyclopentadec-7/8-enylcarbaldehyd 1%.
Schnitt F2: Cyclopentadec-7/8-enylcarbaldehyd (50%)

### Beispiel 4: Cu-katalysierte oxidative Decarbonylierung des Schnitts F2 mit hohem Anteil an Cyclopentadecenylcarbaldehyden.

10 g eines Gemisches F2 aus Beispiel 3, im Wesentlichen bestehend aus 30% Cyclohexadec-8-enon (V) und 50% Cyclopentadecenylcarbaldehyd (VI) wurden in 30 ml DMF gelöst. Zu der Mischung wurden 1.93 g DBU (12.7 mmol, 0.6 eq) hinzugegeben. Durch die Mischung wurde kontinuierlich ein Luftstrom geleitet. 2,2'-Bipyridin (0.06 g, 0.4 mmol, 2 mol%) und Cu(OAc)₂ (0.08 g, 0.4 mmol, 2 mol%) wurden in 5 ml DMF gelöst. Die Cu-bipy-Mischung wurde über eine Spritzenpumpe kontinuierlich über 8 h der Reaktionslösung hinzugetropft. Die Reaktion wurde bei 50 °C für 20 h gerührt. Anschließend wurden 100 ml EtOAc hinzugegeben sowie 100 ml 0.1 M HCl. Die org. Phase wurde extrahiert. Anschließend wurde die wässrige Phase noch einmal mit EtOAc (2 x 50 ml) gewaschen. Die vereinten organischen Phasen würden über Na₂SO₄ getrocknet, abfiltriert und im Vakuum eingeengt. Der Rückstand wurde gaschromatographisch untersucht. Der Umsatz an Carbaldehyd betrug > 95%, die Selektivität zu Cyclopentadecenon lag bei 95%. Der Umsatz an Cyclohexadec-8-enon war 0%.

Das Gemisch bestehend aus Cyclopentadecenon und Cyclohexadecenon wurde säulenchromatographisch mit einem Laufmittel Cyclohexan : EtOAc (30 : 1 → 20 : 1) gereinigt. (Säulematerial: Kieselgel F₂₅₄). Es wurden 1.8 g eines leicht gelblichen Öls erhalten, welches zu 49% Cyclopentadecenon und 43% Cyclohexadecenon enthielt.

Riechtest: schwach nach Moschus, metallisch, aldehydisch, Kunststoffnote

### Beispiel 5: Cu-katalysierte oxidative Decarbonylierung ohne Lösungsmittel

400 g einer Mischung bestehend aus 81% (E/Z)-Cyclohexadec-8-enon (V) und 5.9% Cyclopentadec-8-enylcarbaldehyd (VI) wurden mit 18.5 g Diazabicycloundecen (DBU, 121.8 mmol, 0.6 eq), 0.6 g TMEDA (5.1 mmol, 2.5 mol%) und 0.92 g Cu(OAc)₂ (5.1 mmol, 2.5 mol%) versetzt. Das Reaktionsgemisch wurde auf 50 °C erwärmt und durch die Mischung wurde kontinuierlich ein Luftstrom (100 NI/h) geleitet. Die Mischung wurde 4 h gerührt. Anschließend wurden 200 ml EtOAc, 200 ml Wasser und 43.4 g AcOH bei Raumtemperatur hinzugegeben und die Mischung 5 min gerührt. Die Phasen wurden getrennt und die wässrige Phase wurde noch einmal mit 200 ml EtOAc extrahiert. Die vereinten organischen Phasen wurden über Na₂SO₄ getrocknet, abfiltriert und eingeengt. Der Umsatz der Carbaldehyden betrug 97%. Die Selektivität der Cyclopentadecenone lag bei 85%. Der Verlust an Cyclohexadec-8-enon betrug 0.2%.

Alle %-Angaben basieren auf Gew.-%.

Auf die Offenbarung der hierin erwähnten Druckschriften wird ausdrücklich Bezug genommen.

## Patentansprüche

1. Verfahren zur Herstellung von Cyclohexadec-8-en-1-on und Cyclopentadecenon umfassend die folgenden Schritte:
i) Umsetzung von Cyclohexadeca-1,9-dien mit N₂O unter Erhalt eines ersten Reaktionsgemisches (RG1), umfassend Cyclohexadec-8-en-1-on und Cyclopentadecenylcarbaldehyde;
ii) Umsetzung von RG1 mit Sauerstoff in Gegenwart eines Cu-(II)-Katalysators, wobei die enthaltenen Cyclopentadecenylcarbaldehyde durch oxidative Decarbonylierung zu Cyclopentadecenonen überführt werden, wobei man ein zweites Reaktionsgemisch (RG2) erhält; und
iii) Isolierung von Cyclohexadec-8-en-1-on und Cyclopentadecenon aus RG2.

2. Verfahren nach Anspruch 1, wobei man aus RG1 nicht umgesetztes Cyclohexadeca-1,9-dien abtrennt, wobei man ein Reaktionsgemisch RG1a erhält, umfassend Cyclohexadec-8-en-1-on und Cyclopentadecenylcarbaldehyde, wobei RG1a insbesondere weniger als 1% Cyclohexadeca-1,9-dien enthält; und wobei man nicht umgesetztes Cyclohexadeca-1,9-dien gegebenenfalls in die Umsetzung nach Stufe i) zurückführt.

3. Verfahren nach Anspruch 2, wobei man RG1a reinigt, wobei man eine erste Fraktion (F1), angereichert an Cyclohexadec-8-en-1-on, insbesondere mit weniger als 1% Cyclopentadecenylcarbaldehyden, und wenigstens eine zweite Fraktion (F2), angereichert an Cyclopentadecenylcarbaldehyden, insbesondere enthaltend Cyclohexadec-8-en-1-on mit mehr als 10% Cyclopentadecenylcarbaldehyden, erhält.

4. Verfahren nach einem der vorhergehenden Ansprüche, wobei man in Stufe ii) RG1, RG1a oder F2, oder eine Mischung davon, einsetzt.

5. Verfahren nach einem der vorhergehenden Ansprüche, wobei man in Stufe iii) ein Reaktionsgemisch RG2a, erhalten aus der oxidativen Decarbonylierung (Stufe ii)) der FraktionF2, einsetzt.

6. Verfahren nach einem der vorhergehenden Ansprüche, wobei Cyclohexadec-8-en-1-on und aus der oxidativen Decarbonylierung entstandene Cyclopentadecenone in RG2 oder RG2a voneinander destillativ getrennt werden.

7. Verfahren nach einem der vorhergehenden Ansprüche, wobei das in Stufe ii) gebildete Cyclopentadecenon ausgewählt ist unter (E/Z)-Cyclopentadec-8-en-1-on, (E/Z)-Cyclopentadec-7-en-1-on, einzelnen Stereoisomeren davon und Mischungen solcher Stereoisomere.

8. Verfahren nach einem der vorhergehenden Ansprüche, wobei man den Cu-(II)-Katalysator aus Stufe ii) in situ bildet, indem man zu einem Cu-(II)-Salz einen Liganden gibt.

9. Verfahren nach einem der vorhergehenden Ansprüche, wobei das in Stufe ii) eingesetzte RG1, RG1a oder F2, neben dem (E/Z)-Cyclohexadec-8-en-1-on, (E/Z)-Cyclopentadec-8-enyl-1-carbaldehyd oder eine Mischung aus (E/Z)-Cyclopentadec-8-enyl- und (E/Z)-Cyclopentadec-7-enyl-1-carbaldehyd enthält.

10. Verfahren nach einem der vorhergehenden Ansprüche, wobei man als Cyclopentadecenon wenigstens eine Verbindung, ausgewählt unter (E/Z)-Cyclopentadec-8-en-1-on und (E/Z)-Cyclopentadec-7-en-1-on, erhält.

11. Verfahren nach Anspruch 10, wobei man wenigstens eine Verbindung, ausgewählt unter Verbindungen der folgenden Formeln I, **II, III** und **IV** 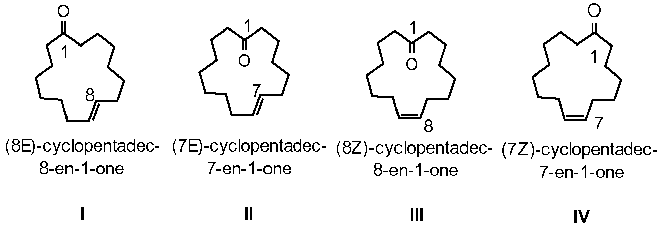 erhält.

12. Verfahren nach einem der vorhergehenden Ansprüche, wobei die Isolierung in Stufe iii) eine Trennung von RG2 in wenigstens eine Fraktion F3, enthaltend Cyclohexadec-8-en-1-on, und wenigstens eine Fraktion F4, enthaltend wenigstens ein Cyclopentadecenon (**I-**IV), durch Destillation und/oder Chromatographie umfasst.

13. Verfahren zur Herstellung einer Riechstoffzusammensetzung, enthaltend wenigstens einen Stoff oder ein Stoffgemisch, ausgewählt unter den oben definierten Fraktionen F1, F3 und F4, oder daraus isolierten Einzelverbindungen, gegebenenfalls in stereoisomerenreiner Form, umfassend die Durchführung eines Verfahrens gemäß den Ansprüchen 1 bis 12.

14. Verfahren zur Herstellung eines Mittels, enthaltend wenigstens einen Stoff oder ein Stoffgemisch, ausgewählt unter den oben definierten Fraktionen F1, F3 und F4, oder daraus isolierten Einzelverbindungen, gegebenenfalls in stereoisomerenreiner Form, umfassend die Durchführung eines Verfahren gemäß den Ansprüchen 1 bis 12.

15. Verfahren zur Reinigung von Cyclohexadec-8-en-1-on, wobei man ein wie in einem der Verfahrensansprüche 1 bis 3 definiertes Gemisch RG1 oder RG1a oder F2 einer oxidativen Decarbonylierung, definiert wie in einem der Verfahrensansprüche 1 bis 12, unterzieht und Cyclohexadec-8-en-1-on von den durch oxidierten Decarbonylierung gebildeten Produkten, insbesondere destillativ und / oder chromatographisch trennt.

## Claims

1. A method for preparing cyclohexadec-8-en-1-one and cyclopentadecenone comprising the following stages:
i) reacting cyclohexadeca-1,9-diene with N₂O to obtain a first reaction mixture (RM1) comprising cyclohexadec-8-en-1-one and cyclopentadecenyl carbaldehydes;
ii) reacting RM1 with oxygen in the presence of a Cu(II) catalyst, wherein the cyclopentadecenyl carbaldehydes present are converted by oxidative decarbonylation to cyclopentadecenones, whereupon a second reaction mixture (RM2) is obtained; and
iii) isolating cyclohexadec-8-en-1-one and cyclopentadecenone from RM2.

2. The method according to claim 1, wherein unreacted cyclohexadeca-1,9-diene is removed from RM1, whereupon a reaction mixture RMla is obtained comprising cyclohexadec-8-en-1-one and cyclopentadecenyl carbaldehydes, where RMla in particular comprises less than 1% cyclohexadeca-1,9-diene; and wherein unreacted cyclohexadeca-1,9-diene is optionally fed back into the reaction after stage i).

3. The method according to claim 2, wherein RMla is purified, whereupon a first fraction (F1) enriched in cyclohexadec-8-en-1-one is obtained particularly comprising less than 1% cyclopentadecenyl carbaldehydes, and at least one second fraction (F2) enriched in cyclopentadecenyl carbaldehydes is obtained particularly comprising cyclohexadec-8-en-1-one with more than 10% cyclopentadecenyl carbaldehydes.

4. The method according to any of the preceding claims, wherein RM1, RMla or F2, or a mixture thereof, is used in stage ii).

5. The method according to any of the preceding claims, wherein a reaction mixture RM2a, obtained from the oxidative decarbonylation (stage ii)) of fraction F2, is used in stage iii).

6. The method according to any of the preceding claims, wherein cyclohexadec-8-en-1-one and cyclopentadecenones obtained from the oxidative decarbonylation in RM2 or RM2a are separated from each other by distillation.

7. The method according to any of the preceding claims, wherein the cyclopentadecenone formed in stage ii) is selected from (E/Z)-cyclopentadec-8-en-1-one, (E/Z)-cyclopentadec-7-en-1-one, individual stereoisomers thereof and mixtures of such stereoisomers.

8. The method according to any of the preceding claims, wherein the Cu(II) catalyst in stage ii) is formed in situ by adding a ligand to a Cu(II) salt.

9. The method according to any of the preceding claims, wherein RM1, RMla or F2 used in stage ii) comprises, in addition to (E/Z)-cyclohexadec-8-en-1-one, (E/Z)-cyclopentadec-8-enyl-1-carbaldehyde or a mixture of (E/Z)-cyclopentadec-8-enyl-1-carbaldehyde and (E/Z)-cyclopentadec-7-enyl-1-carbaldehyde.

10. The method according to any of the preceding claims, wherein the cyclopentadecenone obtained is at least one compound selected from (E/Z)-cyclopentadec-8-en-1-one and (E/Z)-cyclopentadec-7-en-1-one.

11. The method according to claim 10, wherein at least one compound is obtained selected from compounds of the following formulae **I, II, III** and **IV**

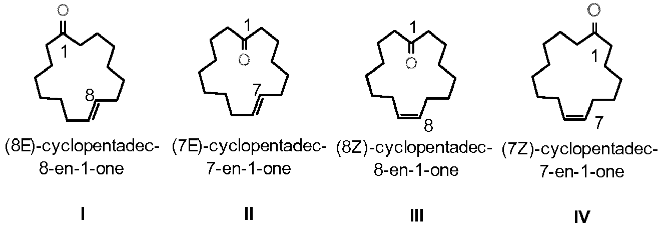

12. The method according to any of the preceding claims, wherein the isolation in stage iii) comprises a separation of RM2 by distillation and/or chromatography into at least one fraction F3, comprising cyclohexadec-8-en-1-one and at least one, fraction F4, comprising at least one cyclopentadecenone (**I-IV**).

13. A method for producing a fragrance composition comprising at least one substance or one substance mixture selected from the fractions F1, F3 and F4 defined above, or individual compounds isolated therefrom, optionally in stereoisomerically pure form, comprising the performance of a method according to claims 1 to 12.

14. A method for producing an agent comprising at least one substance or one substance mixture selected from the fractions F1, F3 and F4 defined above, or individual compounds isolated therefrom, optionally in stereoisomerically pure form, comprising the performance of a method according to claims 1 to 12.

15. A method for purifying cyclohexadec-8-en-1-one, wherein a mixture RM1 or RMla or F2 as defined in any of the method claims 1 to 3 is subjected to an oxidative decarbonylation as defined in any of method claims 1 to 12, and cyclohexadec-8-en-1-one is separated from the oxidized decarbonylation products formed, particularly by distillation and/or chromatographically.

## Revendications

1. Procédé de fabrication de cyclohexadéc-8-én-1-one et de cyclopentadécénone, comprenant les étapes suivantes :
i) la mise en réaction de cyclohexadéca-1,9-diène avec du N₂O pour obtenir un premier mélange réactionnel (RG1), comprenant de la cyclohexadéc-8-én-1-one et des cyclopentadécénylcarbaldéhydes ;
ii) la mise en réaction de RG1 avec de l'oxygène en présence d'un catalyseur de Cu(II), les cyclopentadécénylcarbaldéhydes contenus étant transformés par décarbonylation oxydative en cyclopentadécénones, un deuxième mélange réactionnel (RG2) étant obtenu ; et
iii) l'isolement de cyclohexadéc-8-én-1-one et de cyclopentadécénone à partir de RG2.

2. Procédé selon la revendication 1, dans lequel le cyclohexadéca-1,9-diène non réagi est séparé de RG1, un mélange réactionnel RG1a étant obtenu, comprenant de la cyclohexadéc-8-én-1-one et des cyclopentadécénylcarbaldéhydes, RG1a contenant notamment moins de 1 % de cyclohexadéca-1,9-diène ; et le cyclohexadéca-1,9-diène non réagi étant éventuellement recyclé dans la réaction selon l'étape i) .

3. Procédé selon la revendication 2, dans lequel RG1a est purifié, une première fraction (F1), enrichie en cyclohexadéc-8-én-1-one, notamment avec moins de 1 % de cyclopentadécénylcarbaldéhydes, et au moins une deuxième fraction (F2), enrichie en cyclopentadécénylcarbaldéhydes, contenant notamment de la cyclohexadéc-8-én-1-one avec plus de 10 % de cyclopentadécénylcarbaldéhydes, étant obtenues.

4. Procédé selon l'une quelconque des revendications précédentes, dans lequel RG1, RG1a ou F2 ou un mélange de ceux-ci est utilisé à l'étape ii).

5. Procédé selon l'une quelconque des revendications précédentes, dans lequel un mélange réactionnel RG2a, obtenu par la décarbonylation oxydative (étape ii)) de la fraction F2 est utilisé à l'étape iii).

6. Procédé selon l'une quelconque des revendications précédentes, dans lequel la cyclohexadéc-8-én-1-one et les cyclopentadécénones formées par la décarbonylation oxydative sont séparées par distillation les unes des autres dans RG2 ou RG2a.

7. Procédé selon l'une quelconque des revendications précédentes, dans lequel la cyclopentadécénone formée à l'étape ii) est choisie parmi la (E/Z)-cyclopentadéc-8-én-1-one, la (E/Z)-cyclopentadéc-7-én-1-one, les stéréoisomères individuels de celles-ci et les mélanges de tels stéréoisomères.

8. Procédé selon l'une quelconque des revendications précédentes, dans lequel le catalyseur de Cu(II) est formé in situ à l'étape ii) par ajout d'un ligand à un sel de Cu(II).

9. Procédé selon l'une quelconque des revendications précédentes, dans lequel le RG1, RG1a ou F2 utilisé à l'étape ii) contient, outre la (E/Z)-cyclohexadéc-8-én-1-one, du (E/Z)-cyclopentadéc-8-ényl-1-carbaldéhyde ou un mélange de (E/Z)-cyclopentadéc-8-ényl- et (E/Z)-cyclopentadéc-7-ényl-1-carbaldéhyde.

10. Procédé selon l'une quelconque des revendications précédentes, dans lequel au moins un composé choisi parmi la (E/Z)-cyclopentadéc-8-én-1-one et la (E/Z)-cyclopentadéc-7-én-1-one est obtenu en tant que cyclopentadécénone.

11. Procédé selon la revendication 10, dans lequel au moins un composé, choisi parmi les composés des formules **I, II, III** et **IV** suivantes : 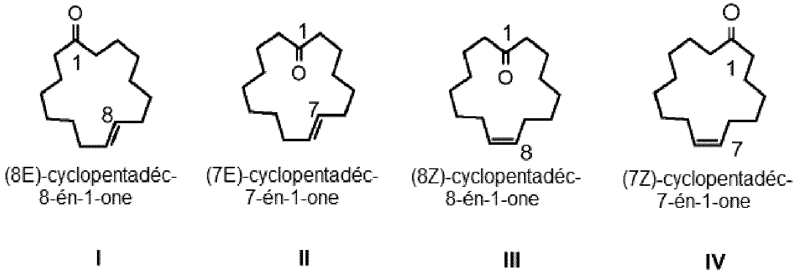 est obtenu.

12. Procédé selon l'une quelconque des revendications précédentes, dans lequel l'isolement à l'étape iii) comprend une séparation de RG2 en au moins une fraction F3, contenant de la cyclohexadéc-8-én-1-one, et au moins une fraction F4, contenant au moins une cyclopentadécénone (**I-IV**), par distillation et/ou chromatographie.

13. Procédé de fabrication d'une composition de matière odorante, contenant au moins une matière ou un mélange de matières, choisies parmi les fractions F1, F3 et F4 définies précédemment, ou des composés individuels isolés à partir de celles-ci, éventuellement sous forme stéréoisomériquement pure, comprenant la réalisation d'un procédé selon les revendications 1 à 12.

14. Procédé de fabrication d'un agent, contenant au moins une matière ou un mélange de matières, choisies parmi les fractions F1, F3 et F4 définies précédemment, ou des composés individuels isolés à partir de celles-ci, éventuellement sous forme stéréoisomériquement pure, comprenant la réalisation d'un procédé selon les revendications 1 à 12.

15. Procédé de purification de cyclohexadéc-8-én-1-one, dans lequel un mélange RG1 ou RG1a ou F2 tel que défini dans l'une quelconque des revendications de procédé 1 à 3 est soumis à une décarbonylation oxydative, telle que définie dans l'une quelconque des revendications de procédé 1 à 12, et la cyclohexadéc-8-én-1-one est séparée des produits formés par la décarbonylation oxydative, notamment par distillation et/ou chromatographie.
